# EUROPÄISCHE PATENTSCHRIFT

(11) **EP 2 049 581 B1**
(45) Veröffentlichungstag und Bekanntmachung des Hinweises auf die Patenterteilung: **20.04.2011**
(21) Anmeldenummer: 07764637.0
(22) Anmeldetag: 13.06.2007
(51) Int. Cl.: C08G 18/08, C08G 18/12, C08G 18/48, C08G 18/50, C08G 18/66

(54) **POLYETHERAMIN-MAKROMONOMERE MIT ZWEI BENACHBARTEN HYDROXYLGRUPPEN UND IHRE VERWENDUNG ZUR HERSTELLUNG VON POLYURETHANEN**
POLYETHERAMINE MACROMONOMERS COMPRISING TWO NEIGHBORING HYDROXYL GROUPS AND THEIR USE FOR PRODUCING POLYURETHANES
MACROMONOMÈRES DE POLYÉTHERAMINE AYANT DEUX GROUPES HYDROXYLE VOISINS ET LEUR UTILISATION POUR LA FABRICATION DE POLYURÉTHANNES

(30) Priorität: 03.08.2006 DE 102006036220
(43) Veröffentlichungstag der Anmeldung: 22.04.2009
(73) Patentinhaber: Clariant Finance (BVI) Limited, Road Town, Tortola (VG)
(72) Erfinder: PÖLLMANN, Klaus, 81475 München (DE); MÜNTER, Jürgen, 70736 Fellbach (DE)
(74) Vertreter: Mikulecky, Klaus
(86) Internationale Anmeldenummer: PCT/EP2007/005207
(87) Internationale Veröffentlichungsnummer: WO 2008/014844

(56) Entgegenhaltungen:
- WO-A-2006/084592
- SCHULTZ R A ET AL: "12-, 15-, and 18-Membered-Ring Nitrogen-Pivot Lariat Ethers: Syntheses, Properties, and Sodium and Ammonium Cation Binding Properties" JOURNAL OF THE AMERICAN CHEMICAL SOCIETY, AMERICAN CHEMICAL SOCIETY, WASHINGTON, DC, US, Bd. 107, 1985, Seiten 6659-6668, XP002377502 ISSN: 0002-7863

## Beschreibung

Die vorliegende Erfindung betrifft Ω-(Alkoxy)-α-N,N-Dihydroxyalkylamino-Polyalkylenglykole, und ihre Verwendung zur Herstellung von wasserdispergierbaren Polyurethanen.

Polyurethansysteme haben sich durch ihre hohe Beständigkeit und einfache Applikation ein weites Anwendungsfeld im Bereich Farben-, Lack-, Coating- und Textilindustrie erschlossen. Aus Gründen des Umwelt- und Arbeitsschutzes wurden in der jüngeren Vergangenheit vor allem lösungsmittelfreie, wasserdispergierte Polyurethansysteme entwickelt.

Die-Herstellung von wässrigen Polyurethan-Dispersionen ist seit vielen Jahren bekannt und wird in einer großen Zahl von Veröffentlichungen im Detail beschrieben (z.B. Houben-Weyl, Methoden der Organischen Chemie, Band E20, Teil I, S. 1659-1681; D. Dieterich, Prog. Org. Coat. 1981, 9, 281-330; J.W. Rosthauser, K. Nachtkamp, Journal of Coated Fabrics 1986, 16, 39-79; R. Arnoldus, Surf. Coat. 1990, 3 (Waterbome Coat.), 179-198).

Wässrige Polyurethan-Dispersionen bestehen aus Polyurethan-Polymeren bzw. Polyurethan-Polyharnstoff-Polymeren, die sowohl Urethan-Gruppen als auch Harnstoff-Gruppen beinhalten und durch Polyadditions-Reaktionen von Polyolen, Polyisocyanaten und Polyaminen zugänglich sind. Aus den Polyolen und den Polyisocyanaten werden zunächst Polyurethan-Prepolymere hergestellt, die dann in der wässrigen Phase dispergiert und mit Polyaminen unter Aufbau der Polyurethan-Polyharnstoff-Polymere kettenverlängert werden. Die PolyurethanPolymere müssen zudem eine ausreichende Menge an hydrophilen Gruppen enthalten, welche die Stabilisierung in der wässrigen Phase gewährleisten. Bei diesen hydrophilen Gruppen handelt es sich um anionische, kationische oder nicht-ionische Gruppen oder eine Kombination der eben genannten Gruppen. Um auf die Verwendung externer Emulgatoren verzichten zu können, ist die Herstellung stabiler wässriger Polyurethandispersionen nur mit Hilfe geeigneter Comonomere möglich, die durch ihre Hydrophilie eine stabile wässrige Dispersion von Polyurethanprepolymeren ermöglichen. (S. Dedrichs, European Coating Journal S. 565,5, 2002, Noll, DE-A-25 51 094). Um einen vollständigen Einbau der hydrophilen Comonomere in das hydrophobe Polyurethanprepolymer zu erreichen, setzt man hierzu Diole mit hydrophilen Resten wie z.B. Dimethylolpropansäure (DMPA) ein. Die Stabilisierung der Dispersion erfolgt in diesem Fall nach Neutralisation der Carbonsäureseitengruppen durch elektrostatische Abstoßung der ins Prepolymer eingebauten Carboxylatgruppen. (H. Kager, Dissertation, Uni Hamburg 2002, Jung-Eun Yang, Journal of Applied Polymer Science 86, 9 , S.2375).

Neben der elektrostatischen Stabilisierung durch Carboxylatgruppen werden wässrige Polyurethandispersionen auch durch nichtionische, hydrophile, sterisch stabilisierend wirkende Gruppen hergestellt. Zur Erreichung dieser sterischen Stabilisierung von Polyurethandispersionen benötigt man daher lange, hydrophile, nicht mit Isocyanaten reagierende Seitenketten, die wie DMPA über zwei Hydroxylgruppen in das Polyurethanprepolymer eingebaut werden können (DE-A-25 51 094). Als besonders geeignet haben sich dabei Polyether (Polyalkylenglykole) mit zwei freien nahe benachbarten Hydroxylgruppen am gleichen Molekülende und einer langen mit einem Alkoxyende versehenen Polyalkylenseitenkette gemäß folgender Formel 1 erwiesen. (S. Dedrichs, European Coating Journal S. 565,5, 2002, DE 30 49 746 A1).

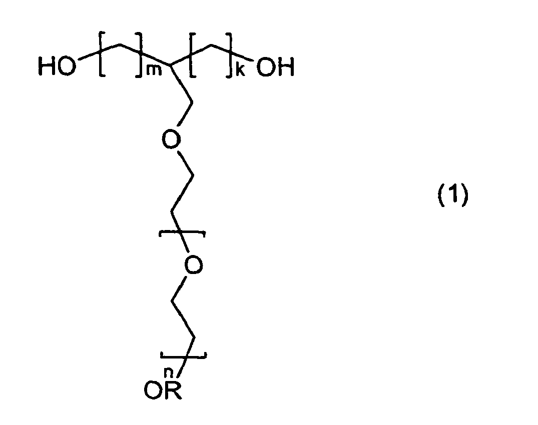

Hierin bedeuten m=k=1; n>=20 und R = Alkyl oder Alkyl-N-C=O

Die Herstellung derartiger Systeme ist jedoch sehr aufwändig und teuer, und verläuft über 4 Stufen ausgehend von trifunktionellen Alkoholen wie Glycerin oder Trimethylolpropan (DE-A-30 49 746, EP-A-0 043 966):
- Stufe 1:: Herstellung eines Hydroxymethyl-1,3-Dioxolans aus dem trifunktionellen Alkohol
- Stufe 2:: Umsetzung des alkalischen Hydroxymethyl-1,3-Dioxolans mit einem Alkylenoxid
- Stufe 3:: Umsetzung des Ω-Hydroxy-α-(1,3-Dioxolano)-Polyalkylenglykols mit einem Alkylhalogenid oder einem Alkylmonoisocyanat
- Stufe 4:: Säurekatalysierte Spaltung des 1,3-Dioxolanrings zum Diol.

Nachteilig bei diesem Verfahren ist, dass große Mengen Alkalihalogenide bei der Veretherung in Stufe 3 als Nebenprodukt anfallen, die die weitere Umsetzung zu Polyurethanprepolymeren stören und schwierig zu entfernen sind. Darüber hinaus können im Falle einer unvollständigen Umsetzung in Stufe 3 neben den Ziel-Diolen, trihydroxy-funktionelle Polyalkylenglykole als Nebenprodukte entstehen, die bei der Einpolymerisation in die Polyurethanprepolymerdispersion Vernetzung und damit Unlöslichkeiten verursachen können (EP-A-00 43 966). Bei der in DE-A-25 14 513 beschriebenen Herstellung von Diisocyanaten mit Polyalkylenglykolseitenketten durch Umsetzung von Triisocyanate mit einem Monohydroxy-funktionellen Polyalkylenglykol ergibt sich das Problem des Entstehens von vernetzend wirkenden Triisocyanate bzw. des Vorhandenseins von Monohydroxy- oder Monoisocyanat-funktionellen Komponenten je nach gewählter Stöchiometrie. Auch hier verursachen Triisocyanat-funktionelle Komponenten potentiell Vernetzung, Monohydroxy- oder Monoisocyanat-funktionellen Komponenten verursachen jedoch Kettenabbruch der linearen PU-Polymere.

Aufgabe der vorliegenden Erfindung war es deshalb, ein einfach und kostengünstig herzustellendes Ω-(Alkoxy)-α-Dihydroxyalkyl-Polyalkylenglykol zu finden, das die oben genannten Nachteile bei der Herstellung und Anwendung nicht aufweist.

Das Ω-(Alkoxy)-α-Dihydroxyalkyl-Polyalkylenglykol sollte in geeigneter Weise in Polyurethanprepolymerdispersionen eingebaut werden können und die Stabilität der wässrigen Prepolymerdispersionen gewährleisten.

Gegenstand der Erfindung sind somit Verbindungen der Formel 2 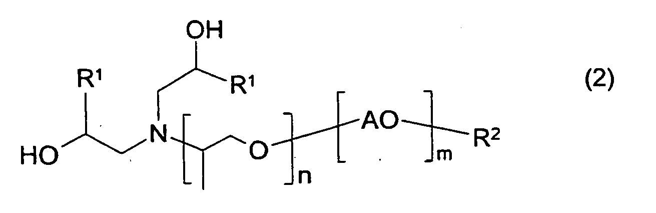 worin
- R¹: H, Methyl oder Ethyl,
- R²: C₁- bis C₄-Alkyl,
- A: eine C₂- bis C₄-Alkylengruppe,
- m: eine Zahl von 1 bis 400
- n: 1, 2, 3, 4, oder 5 bedeuten.

Ein weiterer Gegenstand der Erfindung ist ein Verfahren zur Herstellung von Polyurethan-Prepolymeren, indem man Verbindungen der Formel 2 mit einem Polyisocyanat und gegebenenfalls mit weiteren Polyolen oder Polyaminen umsetzt.

Ein weiterer Gegenstand der Erfindung ist ein Verfahren zur Herstellung von Polyurethanpolymeren, indem man
a) eine Verbindung der Formel 2 mit einem Polyisocyanat und gegebenenfalls mit weiteren Polyolen oder Polyaminen zu einem Polyurethan-Prepolymer umsetzt, und
b) das so erhaltene Polyurethan-Prepolymer in wässrigem Milieu mit einem Polyamin zu einem Polyurethanpolymer umsetzt.

Ein weiterer Gegenstand der Erfindung sind Polyurethan-Prepolymere, erhältlich durch die Reaktion einer Verbindung der Formel 2 mit einem Isocyanat der Formel X(NCO)ₚ, worin p eine Zahl von 2 bis 4 und X ein aliphatischer, cycloaliphatischer, aromatischer oder araliphatischer Kohlenwasserstoffrest ist.

Ein weiterer Gegenstand der Erfindung sind Polyurethanpolymere, erhältlich durch die Reaktion einer Verbindung der Formel 2 mit einem Isocyanat der Formel X(NCO)ₚ, worin p eine Zahl von 2 bis 4 und X ein aliphatischer, cycloaliphatischer, aromatischer oder araliphatischer Kohlenwasserstoffrest ist, und die anschließende Umsetzung des so erhaltenen Polyurethanprepolymers in wässrigem Milieu mit einem Polyamin der Formel Y(NH₂)_{q}, worin Y ein aliphatischer, cycloaliphatischer, aromatischer oder araliphatischer Kohlenwasserstoffrest, und q eine Zahl von 2 bis 4 ist.

Ein weiterer Gegenstand der Erfindung ist die Verwendung der Verbindungen der Formel 2 zur Herstellung von Polyurethan-Prepolymeren, indem die Verbindung der Formel 2 mit einem Isocyanat der Formel X(NCO)ₚ, worin p eine Zahl von 2 bis 4 und X ein aliphatischer, cycloaliphatischer, aromatischer oder araliphatischer Kohlenwasserstoffrest ist, zur Reaktion gebracht wird.

Ein weiterer Gegenstand der Erfindung ist die Verwendung der Verbindungen der Formel 2 zur Herstellung von Polyurethanpolymeren, indem die Verbindung der Formel 2 mit einem Isocyanat der Formel X(NCO)ₚ, worin p eine Zahl von 2 bis 4 und X ein aliphatischer, cycloaliphatischer, aromatischer oder araliphatischer Kohlenwasserstoffrest ist, zur Reaktion gebracht wird, und das so erhaltene Polyurethanprepolymer in wässrigem Milieu mit einem Polyamin der Formel Y(NH₂)_{q}, worin Y ein aliphatischer, cycloaliphatischer, aromatischer oder araliphatischer Kohlenwasserstoffrest, und q eine Zahl von 2 bis 4 ist, umgesetzt wird.

In der durch (A-O)ₘ wiedergegebenen Oxalkylengruppe liegt die Gesamtzahl von Oxalkyleneinheiten vorzugsweise zwischen 3 und 250, insbesondere zwischen 5 und 200. Bei der Oxalkylenkette kann es sich um eine Homopolymer- oder Blockcopolymerkette handeln, die alternierende Blöcke verschiedener Oxalkyleneinheiten aufweist. Es kann sich dabei auch um eine Kette mit statistischer Abfolge der Oxalkyleneinheiten handeln oder um eine Kette mit statistischen und blockartigen Kettenabschnitten. Die Oxalkyleneinheiten sind vorzugsweise entweder nur Oxethyleneinheiten, oder eine Mischung aus Oxethylen- und Oxpropyleneinheiten, wobei vorzugsweise mindestens 50 mol-% der Reste (A-O) Oxethylenreste sind.

In einer weiteren bevorzugten Ausführungsform steht -(A-O)ₘ-R² für eine Oxalkylenkette der Formel

- (CH₂-CH₂-O)_{b} - (CH(CH₃)-CH₂-O)ₐ - R²

worin
- a: eine Zahl von 0 bis 300, vorzugsweise 1 bis 50
- b: eine Zahl von 3 bis 300, vorzugsweise 5 bis 200
und R² die oben angegebene Bedeutung hat.
Vorzugsweise steht R¹ für Wasserstoff.
Vorzugsweise steht R² für Methyl.

In einer weiteren bevorzugten Ausführungsform stehen R¹ für Wasserstoff und R² für Methyl.

In einer weiteren bevorzugten Ausführungsform ist n gleich 2, 3 oder 4.

Die Verbindungen der Formeln 2 werden im Folgenden auch als Ω-Alkoxy-Polyetheramindiole bezeichnet.

Im Folgenden wird das Verfahren zur Herstellung der Ω-Alkoxy-Polyetheramindiole sowie die Herstellung von Polyurethan-Dispersionen damit näher beschrieben und an Beispielen erläutert.

Die Ω-Alkoxy-Polyetheramindiole können aus kommerziell verfügbaren α-Amino-Ω-Alkoxypolyalkylengiykolen hergestellt werden (DE-A-16 43 426), oder man verwendet speziell für diesen Zweck hergestellte α-Amino-Ω-Alkoxypolyalkylenglykolen die aus α-Hydroxy-Ω-Alkoxypolyalkylenglykolen nach dem in DE-A-16 43 426 beschriebenen Verfahren hergestellt werden, indem in einer Aminolysereaktion die α-Hydroxy-Gruppe durch eine primäre Aminogruppe ausgetauscht wird. Diese primäre Aminogruppe wird anschließend ohne Zugabe eines Alkoxylierungskatalysators mit genau 2 Mol Alkylenoxid zur Dihydroxyalkylaminogruppe umgesetzt.

Der Grad der Wasserlöslichkeit; definiert durch den Trübungspunkt nach DIN EN 1890, der Ω-Alkoxy-Polyetheramindiofe, sowie der Grad ihrer hydrophilierenden und dispergierenden Wirkung kann durch das Verhältnis und Anzahl der Oxalkyleneinheiten (AO)ₘ, vorzugsweise von Ethylenoxid zu Propylenoxid, eingestellt werden.

Die lsocyanate der Formel X(NCO)ₚ sind vorzugsweise solche, in denen X für einen aliphatischen Kohlenwasserstoffrest mit 4 bis 12 Kohlenstoffatomen, einen cycloaliphatischen oder aromatischen Kohlenwasserstoffrest mit 6 bis 15 Kohlenstoffatomen oder einen araliphatischen Kohlenwasserstoffrest mit 7 bis 15 Kohlenstoffatomen steht.

Beispiele derartiger Diisocyanate sind Tetramethylendiisocyanat, Hexamethylendiisocyanat, Dodecamethylendiisocyanat, 1,4-Diisocyanatocyclohexan, 1-Isocyanato-3,5,5-trimethyl-5-isocyanatomethylcyclohexan (IPDI), 2,2-Bis-(4-isocyanatöcyclohexyl)-propan, Trimethylhexandiisocyanat, 1,4-Diisocyanatobenzol, 2,4-Diisocyanatotoluol, 2,6-Diisocyanatotoluol, 4,4'-Diisocyanato-diphenylmethan, 2,4'-Diisocyanato-diphenylmethan, p-Xylylendiisocyanat, Tetramethylxylylendüsocyanat (TMXDI), die Isomeren des Bis-(4-isocyanatocyclohexyl)methans (HMDI) wie das trans/trans-, das cis/-cis-und das cis/trans-Isomere sowie aus diesen Verbindungen bestehende Gemische.

Als Gemische dieser Isocyanate sind besonders die Mischungen der jeweiligen Strukturisomeren von Diisocyanattoluol und Diisocyanato-diphenylmethan von Bedeutung, insbesondere ist die Mischung aus 80 mol-% 2,4-Diisocyanatotoluol und 20 mol-% 2,6-Diisocyanatotoluol geeignet. Weiterhin sind die Mischungen von aromatischen Isocyanaten wie 2,4 Diisocyanattoluol und/oder 2,6-Diisocyanatotoluol mit aliphatischen oder cycloaliphatischen Isocyanaten wie Hexamethylendiisocyanat oder IPDI besonders vorteilhaft, wobei das bevorzugte Mischungsverhältnis der aliphatischen zu aromatischen Isocyanate 4 : 1 bis 1 : 4 beträgt.

Zum Aufbau der Polyurethane kann man als Verbindungen außer den vorgenannten auch Isocyanate einsetzen, die neben den freien Isocyanatgruppen weitere verkappte Isocyanatgruppen, z.B. Uretdiongruppen tragen.

Die für die Umsetzung der Polyurethan-Prepolymere zu den Polyurethan-Polymeren zum Einsatz kommenden Polyamine sind solche, in denen Y für einen aliphatischen Kohlenwasserstoffrest mit 4 bis 12 Kohlenstoffatomen, einen cycloaliphatischen oder aromatischen Kohlenwasserstoffrest mit 6 bis 15 Kohlenstoffatomen oder einen araliphatischen Kohlenwasserstoffrest mit 7 bis 15 Kohlenstoffatomen steht. Bevorzugte Amine sind polyfunktionelle Amine des Molgewichtsbereichs von 32 bis 500 g/mol, vorzugsweise von 60 bis 300 g/mol, welche mindestens zwei Aminogruppen, ausgewählt aus der Gruppe der primären und sekundären Aminogruppen, enthalten. Beispiele hierfür sind Diamine wie Diaminoethan, Diaminopropane, Diaminobutane, Diaminohexane, Piperazin, 2,5-Dimethylpiperazin, Amino-3-aminomethyl-3,5,5-trimethylcyclohexan (Isophorondiamin, IPDA), 4,4'-Diaminodicyclohexylmethan, 1,4-Diaminocyclohexan, Aminoethylethanolamin, Hydrazin, Hydrazinhydrat oder Triamine wie Diethylentriamin oder 1,8-Diamino-4-aminomethyloctan.

Die Amine können auch in blockierter Form, z.B. in Form der entsprechenden Ketimine (siehe z.B. CA-A-1 129 128), Ketazine (vgl. z.B. die US-4 269 748) oder Aminsalze (s. US-4 292 226) eingesetzt werden. Auch Oxazolidine, wie sie beispielsweise in der US-4 192 937 verwendet werden, stellen verkappte Polyamine dar, die für die Herstellung der erfindungsgemäßen Polyurethane zur Kettenverlängerung der Präpolymeren eingesetzt werden können. Bevorzugt werden Gemische von Di- und Triaminen verwendet, besonders bevorzugt Gemische von Isophorondiamin (IPDA) und Diethylentriamin (DETA).

Die beschriebenen Polyamine sind ebenfalls zur Verwendung bei der Umsetzung der Verbindung der Formel 2 mit einem Polyisocyanat geeignet.

Gegebenenfalls werden bei der Umsetzung der Verbindungen der Formel 2 mit Polyisocyanaten zum Polyurethan-Prepolymer, und bei der Herstellung der Polyurethanpolymere Diole eingesetzt.

Bei den Diolen handelt es sich insbesondere um Polyesterpolyole, die z.B. aus Ullmanns Encyklopädie der technischen Chemie, 4. Auflage, Band 19, S. 62 bis 65 bekannt sind. Bevorzugt werden Polyesterpolyole eingesetzt, die durch Umsetzung von zweiwertigen Alkoholen mit zweiwertigen Carbonsäuren erhalten werden. Anstelle der freien Polycarbonsäuren können auch die entsprechenden Polycarbonsäureanhydride oder entsprechende Polycarbonsäureester von niederen Alkoholen oder deren Gemische zur Herstellung der Polyesterpolyole verwendet werden. Die Polycarbonsäuren können aliphatisch, cycloaliphatisch, araliphatisch, aromatisch oder heterocyclisch sein und gegebenenfalls, z.B. durch Halogenatome, substituiert und/oder ungesättigt sein. Als Beispiele hierfür seien genannt:
Korksäure, Azelainsäure, Phthalsäure, Isophthalsäure, Phthalsäureanhydrid, Tetrahydrophthalsäureanhydrid, Hexahydrophthalsäureanhydrid, Tetrachlorphthalsäureanhydrid, Endomethylentetrahydrophthalsäureanhydrid, Glutarsäureanhydrid, Maleinsäure, Maleinsäureanhydrid, Fumarsäure, dimere Fettsäuren. Bevorzugt sind Dicarbonsäuren der allgemeinen Formel HOOC-(CH₂)_{y}-COOH, wobei y eine Zahl von 1 bis 20, bevorzugt eine gerade Zahl von 2 bis 20 ist, z.B. Bernsteinsäure, Adipinsäure, Sebacinsäure und Dodecandicarbonsäure.

Als mehrwertige-Alkohole kommen z.B. Ethylenglykol, Propan-1,2-diol, Propan-1,3-diol, Butan-1,3-diol, Buten-1,4-diol, Butin-1,4-diol, Pentan-1,5-diol, Neopentylglykol, Bis-(hydroxymethyl)-cyclohexane wie 1,4-Bis-(hydroxymethyl)cyclohexan, 2-Methyl-propan-1,3-diol, Methylpentandiole, ferner Diethylenglykol, Triethylenglykol, Tetraethylenglykol, Polyethylenglykol, Dipropylenglykol, Polypropylenglykol, Dibutylenglykol und Polybutylenglykole in Betracht.

Bevorzugt sind Alkohole der allgemeinen Formel HO-(CH₂)ₓ-OH, wobei x eine Zahl von 1 bis 20, bevorzugt eine gerade Zahl von 2 bis 20 ist. Beispiele hierfür sind Ethylenglycol, Butan-1,4-diol, Hexan-1,6-diol, Octan-1,8-diol und Dodecan-1,12-diol. Weiterhin bevorzugt ist Neopentylglykol. Ferner kommen auch Polycarbonat-Diole, wie sie z.B. durch Umsetzung von Phosgen mit einem Überschuss von den als Aufbaukomponenten für die Polyesterpolyole genannten niedermolekularen Alkohole erhalten werden können, in Betracht.

Geeignet sind auch Polyesterdiole auf Lacton-Basis, wobei es sich um Homo-oder Mischpolymerisate von Lactonen, bevorzugt um endständige Hydroxylgruppen aufweisende Anlagerungsprodukte von Lactonen an geeignete difunktionelle Startermoleküle handelt. Als Lactone kommen bevorzugt solche in Betracht, die sich von Verbindungen der allgemeinen Formel HO-(CH₂)_{z}-COOH ableiten, wobei z eine Zahl von 1 bis 20 ist und ein H-Atom einer Methyleneinheit auch durch einen C₁- bis C₄-Alkylrest substituiert sein kann. Beispiele sind ε-Caprolacton, ß-Propiolacton, γ-Butyrolacton und/oder Methyl-ε-caprolacton sowie deren Gemische. Geeignete Starterkomponenten sind z.B. die vorstehend als Aufbaukomponente für die Polyesterpolyole genannten niedermolekularen zweiwertigen Alkohole. Die entsprechenden Polymerisate des ε-Caprolactons sind besonders bevorzugt. Auch niedere Polyesterdiole oder Polyetherdiole können als Starter zur Herstellung der Lacton-Polymerisate eingesetzt sein. Anstelle der Polymerisate von Lactonen können auch die entsprechenden, chemisch äquivalenten Polykondensate der den Lactonen entsprechenden Hydroxycarbonsäuren, eingesetzt werden.

Daneben kommen als Monomere Polyetherdiole in Betracht. Sie sind insbesondere durch Polymerisation von Ethylenoxid, Propylenoxid, Butylenoxid, Tetrahydrofuran, Styroloxid oder Epichlorhydrin mit sich selbst, z.B. in Gegenwart von BF₃ oder durch Anlagerung dieser Verbindungen gegebenenfalls im Gemisch oder nacheinander, an Startkomponenten mit reaktionsfähigen Wasserstoffatomen, wie Alkohole oder Amine, z.B. Wasser, Ethylenglykol, Propan-1,2-diol, Propan-1,3-diol, 1,2-Bis(4-hydroxy-diphenyl)-propan oder Anilin erhältlich. Besonders bevorzugt ist Polytetrahydrofuran eines Molekulargewichts von 240 bis 5000, und vor allem 500 bis 4500.

Ebenfalls geeignet sind Polyhydroxyolefine, bevorzugt solche mit 2 endständigen Hydroxylgruppen, z.B. α,-ω-Dihydroxypolybutadien, α,-ω-Dihydroxypolymethacrylester oder α,-ω-Dihydroxypolyacrylester als Monomere. Solche Verbindungen sind beispielsweise aus der EP-A-0 622 378 bekannt. Weitere geeignete Polyole sind Polyacetale, Polysiloxane und Alkydharze.

Folgende Beispiele verdeutlichen die Erfindung näher.

### Beispiel 1

1. Schritt:
Zunächst wurden 120 g Diethylenglykolmonomethylether (1 mol), der durch Destillation sorgfältig gereinigt war, in einem Druckreaktor vorgelegt. Nach Zugabe von 1 g NaOH wurde bei 90°C unter Vakuum getrocknet. Anschließend wurde bei einer Temperatur von 130°C und einem Druck von ca. 6 bar zunächst ein Gemisch aus 290 g (5 mol) Propylenoxid und 1672 g (38 mol) Ethylenoxid zudosiert und nach dessen vollständiger Abreaktion erkennbar am Druckabfall eine Menge von 232 g (4 mol) Propylenoxid dosiert. Nach der Abreaktion des Propylenoxids erkennbar am Druckabfall wurde die Reaktion durch Zugabe von Essigsäure abgestoppt und das Produkt mittels OH-Zahl-Titration und NMR analysiert. Die OH-Zahl betrug 24 mgKOH/g entsprechend einer Molmasse von 2330 g/mol. Die Verteilung der Oxyethylen-, Oxypropylen-Kettenanteile sowie der Methoxy (CH₃O-), primären (-CH₂OH) und sekundären (-CHCH₃OH)-Endgruppen ist aus dem NMR Spektrum ableitbar:

| funktionelle Gruppen | CH₃-O- | OCH₂CH₂O | OCH₂CHCH₃O | -CHCH₃OH | -CH₂OH |
|---|---|---|---|---|---|
| molares Verhältnis, gemessen durch ¹H-Signale | 1 | 38 | 9 | 0,98 | 0,07 |

Schritt 2:
   730 g des α-Hydroxy-Ω-methoxy-polyoxyalkylen-polyoxypropylenblockcopolymers aus Schritt 1 mit einer Molmasse von 2330 g/mol und einem molaren Verhältnis der Oxypropylen- zu den Oxyethyleneinheiten von 19 zu 81 wurden mit Ammoniak und Wasserstoff in Gegenwart eines Ni-haltigen Katalysators zum entsprechenden Amin umgesetzt. Das resultierende primäre Amin hatte einen Gesamtstickstoffgehalt von 0,60 Gew.-%.

Schritt 3:
Das Amin aus Schritt 2 wurde nach Entfernung des Katalysators mit 2 Moläquivalenten Ethylenoxid (34 g) bei 190°C und einem Druck von 4 bar zum entsprechenden α-Dihydroxyethylamino-Ω-methoxy-polyalkylenglykol umgesetzt.
Der Gesamtstickstoffgehalt nach der Umsetzung betrug 0,57 Gew.-%, entsprechend einer Molmasse von 2456 g/mol. Der Anteil an tertiärem Amin lag bei 98,2 Gew.-%. Das Produkt wurde mittels ¹H-NMR charakterisiert.

| funktionelle Gruppen | CH₃-O- | OCH₂CH₂O | OCH₂CHCH₃O | -CH₂N(CH₂CH₂OH)₂ | -CH₂OH |
|---|---|---|---|---|---|
| molares Verhältnis, gemessen durch ¹H-Signale | 1 mol | 39 mol | 8,5 mol | 1,05 mol | 2,1 |

### Beispiel 2

1. Schritt:
Zunächst wurden 180 g Diethylenglykolmonomethylether (1,5 mol), der durch Destillation sorgfältig gereinigt war, in einem Druckreaktor vorgelegt. Nach Zugabe von 1 g NaOH wurde bei 90°C unter Vakuum getrocknet. Anschließend wurde bei einer Temperatur von 140°C und einem Druck von ca. 6 bar zunächst 660 g (15 mol) Ethylenoxid zudosiert und nach dessen vollständiger Abreaktion erkennbar am Druckabfall eine Menge von 262 g (4,5 mol) Propylenoxid dosiert.
Nach der Abreaktion des Propylenoxids erkennbar am Druckabfall wurde die Reaktion durch Zugabe von Essigsäure abgestoppt und das Produkt mittels OH-Zahl-Titration und NMR analysiert.
Die OH-Zahl betrug 75 mgKOH/g entsprechend einer Molmasse von 748 g/mol. Die Verteilung der Oxyethylen-, Oxypropylen-Kettenanteile sowie der Methoxy (CH₃O-), primären (-CH₂OH) und sekundären (-CHCH₃OH)-Endgruppen ist aus dem NMR Spektrum ableitbar:

| funktionelle Gruppen | CH₃-O- | OCH₂CH₂O | OCH₂CHCH₂O | -CHCH₃OH | -CH₂OH |
|---|---|---|---|---|---|
| molares Verhältnis, gemessen durch ¹H-Signale | 1 | 12 | 3 | 0,96 | 0,05 |

2. Schritt:
685 g des α-Hydroxy-Ω-methoxy-polyoxyethylen-polyoxypropylen-blockcopolymers aus Schritt 1 mit einer Molmasse von 748 g/mol wurden mit Ammoniak und Wasserstoff in Gegenwart eines Ni-hattigen Katalysators zum entsprechenden Amin umgesetzt. Das resultierende primäre Amin hatte einen Gesamtstickstoffgehalt von 1,78 Gew.-%.

3. Schritt: Das Amin aus Schritt 2 wurde nach Entfernung des Katalysators mit 2 Moläquivalenten Ethylenoxid (81 g) bei 190°C und einem Druck von 4 bar zum entsprechenden α-dihydroxyethylamino-Ω-Methoxy-polyalkylenglykol umgesetzt. Der Gesamtstickstoffgehalt nach der Umsetzung betrug 1,6 Gew.-%, entsprechend einer Molmasse von 875 g/mol. Der Anteil an tertiärem Amin lag bei 99 Gew.-%. Das Produkt wurde mittels ¹H-NMR charakterisiert.

| funktionelle Gruppen | CH₃-O- | OCH₂CH₂O | OCH₂CHCH₃O | -CH₂N(CH₂CH₂OH)₂ | -CH₂OH |
|---|---|---|---|---|---|
| molares Verhältnis, gemessen durch ¹H-Signale | 1 | 12 | 3 | 1 | 2,05 |

### Beispiel 3: Herstellung einer wässrigen Polyurethandispersion im Aceton-Verfahren

224 g eines Adipinsäure-Diethylenglykol-Polyesterdiols (OH-Zahl 52,6), 1,34 g DMPA, 52,5 g des α-Dihydroxyethylamino-Ω-Methoxy-Polyalkylenglykols aus Beispiel 2, 16,8 g Hexamethylendiisocyanat und 44,2 g Isophorondiisocyanat wurden bei 90°C in zwei Stunden zu einem Polyurethanprepolymer umgesetzt. Der theoretische Rest-NCO-Gehalt betrug 3,10 Gew.-%. Der theoretische, durch Titration bestimmte Messwert lag aufgrund des enthaltenen Amins bei 2,36 Gew.-%. Der im Versuch gemessene NCO-Restgehalt betrug 2,18 Gew.-%. Dem Prepolymer wurden 150 g Aceton zugegeben, mit 1 g Triethylamin neutralisiert, auf Raumtemperatur abkühlt und mit 650 g Wasser dispergiert. Die Kettenverlängerung des wässrig dispergierten Prepolymers erfolgte mit 6,7 g Ethylendiamin, gelöst in 50 g Wasser. Bei einer anschließenden Destillation im Vakuum wurde das Aceton entfernt. Es entstand eine milchig weiße, dünnflüssige und lagerstabile Polyurethandispersion mit einem Feststoffgehalt von 30 Gew.-%, einem pH von 8,0 und einem durchschnittlichen Teilchendurchmesser von 200 nm (gemessen mit Particle Size Analyzer 90 Plus, Brookhaven Instruments).

### Beispiel 4:

### Herstellung einer wässrigen Polyurethandispersion im Prepolymer-lonomer-Verfahren

153 g eines Polypropylenglykols (OH-Zahl 110), 70 g des α-Dihydroxyethylamino-Ω-Methoxy-Pofyalkylenglykols aus Beispiel 2 und 77,4 g Isophorondiisocyanat wurden mit 0,1 g Dibutylzinndilaurat bei 75°C in 2,5 Stunden zu einem Polyurethanprepolymer umgesetzt. Der theoretische Rest-NCO-Gehalt betrug 3,36 Gew.-%. Der theoretische, durch Titration bestimmte Messwert lag aufgrund des enthaltenen Amins bei 2,24 Gew.-%. Der im Versuch gemessene NCO-Restgehalt betrug 2,20 Gew.-%. Das Prepolymer wurde auf 45°C abkühlt und in 650 g Wasser dispergiert. Die Kettenverlängerung des wässrig dispergierten Prepolymers erfolgte mit 7,1 g Ethylendiamin, gelöst in 50 g Wasser. Es entstand eine orange durchscheinende, dünnflüssige und lagerstabile Polyurethandispersion mit einem Feststoffgehalt von 30 Gew.-%, einem pH von 8,5 und einem durchschnittlichen Teilchendurchmesser von 40 nm.

## Patentansprüche

1. Verbindungen der Formel 2 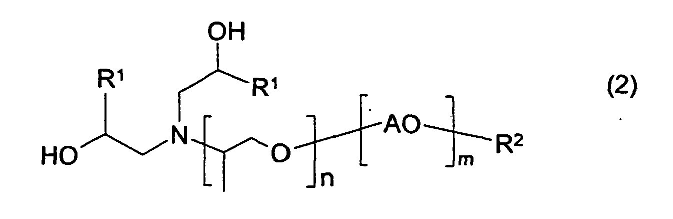 worin
R¹ H, Methyl oder Ethyl,
R² C₁- bis C₄-Alkyl,
A eine C₂- bis C₄-Alkylengruppe,
m eine Zahl von 1 bis 400
n 1, 2, 3, 4, oder 5
bedeuten.

2. Verbindungen gemäß Anspruch 1, worin mindestens 50 mol-% der Reste (A-O) Oxethylenreste -CH₂-CH₂-O- sind.

3. Verbindungen gemäß Anspruch 2, worin die weiteren Reste (A-O) ad 100 mol-% Oxpropylenreste -CH(CH₃)-CH₂-O- sind

4. Verbindungen nach Anspruch 1, worin die (A-O)ₘ-Gruppe für eine reine Oxethylengruppe mit 3 bis 300 Oxethyleneinheiten steht.

5. Verbindungen gemäß einem oder mehreren der Ansprüche 1 bis 4, worin R¹ für H steht.

6. Verbindungen gemäß einem oder mehreren der Ansprüche 1 bis 5, worin R² für CH₃ steht.

7. Verbindungen gemäß einem oder mehreren der Ansprüche 1 bis 6, worin -(A-O)ₘ-R² für eine Oxalkylengruppe der Formel
- (CH₂-CH₂-O)_{b}- (CH(CH₃)-CH₂-O)ₐ-R² steht, worin
a für eine Zahl von 0 bis 300,
b für eine Zahl von 3 bis 300 steht, und
und R² die in Anspruch 1 angegebene Bedeutung hat.

8. Verfahren zur Herstellung von Verbindungen der Formel 2, indem man zunächst ein α-Hydroxy-Ω-Alkoxypolyalkylenglykol mit einer sekundären Hydroxyendgruppe durch Alkoxylierung von R²-OH und anschließender Propoxylierung herstellt, die sekundäre α-Hydroxy-Gruppe anschließend durch eine Aminolyse durch eine primäre Aminogruppe austauscht, und diese primäre Aminogruppe anschließend ohne Zugabe eines Alkoxylierungskatalysators mit 2 Mol Alkylenoxid, insbesondere 2 Mol Ethylenoxid, zur Dihydroxyalkylaminogruppe, insbesondere zur Dihydroxyethylaminogruppe, umsetzt.

9. Verfahren zur Herstellung von Polyurethan-Prepolymeren, indem man Verbindungen der Formel 2 mit einem Polyisocyanat und gegebenenfalls mit weiteren Polyolen oder Polyaminen umsetzt.

10. Verfahren zur Herstellung von Polyurethanpolymeren, indem man
a) eine Verbindung der Formel 2 mit einem Polyisocyanat und gegebenenfalls mit weiteren Polyolen oder Polyaminen zu einem Polyurethan-Prepolymer umsetzt, und
b) das so erhaltene Polyurethan-Prepolymer in wässrigem Milieu mit einem Polyamin zu einem Polyurethanpolymer umsetzt.

11. Polyurethan-Prepolymere, erhältlich durch die Reaktion einer Verbindung der Formel 2 mit einem Isocyanat der Formel X(NCO)p, worin p eine Zahl von 2 bis 4 und X ein aliphatischer, cycloaliphatischer, aromatischer oder araliphatischer Kohlenwasserstoffrest ist.

12. Polyurethanpolymere, erhältlich durch die Reaktion einer Verbindung der Formel 2 mit einem Isocyanat der Formel X(NCO)ₚ, worin p eine Zahl von 2 bis 4 und X ein aliphatischer, cycloaliphatischer, aromatischer oder araliphatischer Kohlenwasserstoffrest ist, und die anschließende Umsetzung des so erhaltenen Polyurethanprepolymers in wässrigem Milieu mit einem Polyamin der Formel Y(NH₂)_{q}, worin Y ein aliphatischer, cycloaliphatischer, aromatischer oder araliphatischer Kohlenwasserstoffrest, und q eine Zahl von 2 bis 4 ist.

13. Verwendung der Verbindungen der Formel 2 zur Herstellung von Polyurethan-Prepolymeren, indem die Verbindung der Formel 2 mit einem Isocyanat der Formel X(NCO)ₚ, worin p eine Zahl von 2 bis 4 und X ein aliphatischer, cycloaliphatischer, aromatischer oder araliphatischer Kohlenwasserstoffrest ist, zur Reaktion gebracht wird.

14. Verwendung der Verbindungen der Formel 2 zur Herstellung von Polyurethanpolymeren, indem die Verbindung der Formel 2 mit einem Isocyanat der Formel X(NCO)p, worin p eine Zahl von 2 bis 4 und X ein aliphatischer, cycloaliphatischer, aromatischer oder araliphatischer Kohlenwasserstoffrest ist, zur Reaktion gebracht wird, und das so erhaltene Polyurethanprepolymer in wässrigem Milieu mit einem Polyamin der Formel Y(NH₂)_{Q}, worin Y ein aliphatischer, cycloaliphatischer, aromatischer oder araliphatischer Kohlenwasserstoffrest, und q eine Zahl von 2 bis 4 ist, umgesetzt wird.

## Claims

1. A compound of formula 2 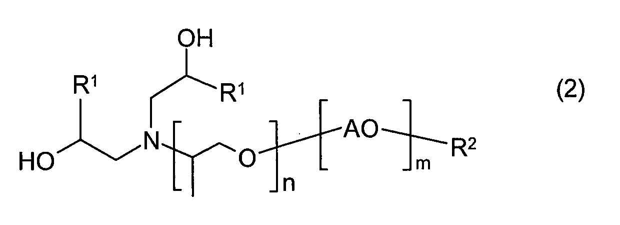 in which
R¹ is H, methyl or ethyl,
R² is C₁- to C₄-alkyl,
A is a C₂- to C₄-alkylene group,
m is a number from 1 to 400
n is 1, 2, 3, 4 or 5.

2. A compound as claimed in claim 1, in which at least 50 mol% of the radicals (A-O) are oxethylene radicals -CH₂-CH₂-O-.

3. A compound as claimed in claim 2, in which the further radicals (A-O) ad 100 mol% are oxpropylene radicals -CH(CH₃)-CH₂-O-.

4. A compound as claimed in claim 1, in which the (A-O)ₘ group is a pure oxethylene group having 3 to 300 oxethylene units.

5. A compound as claimed in one or more of claims 1 to 4, in which R¹ is H.

6. A compound as claimed in one or more of claims 1 to 5, in which R² is CH₃.

7. A compound as claimed in one or more of claims 1 to 6, in which -(A-O)ₘ-R² is an oxalkylene group of the formula
- (CH₂-CH₂-O)_{b}-(CH(CH₃)-CH₂-O)ₐ-R² in which
a is a number from 0 to 300,
b is a number from 3 to 300, and
and R² has the meaning given in claim 1.

8. A process for producing compounds of formula 2, by firstly producing an α-hydroxy-Ω-alkoxypolyalkylene glycol with a secondary hydroxyl end group by alkoxylation of R²-OH and subsequent propoxylation, then replacing the secondary α-hydroxy group through an aminolysis by a primary amino group, and then reacting this primary amino group without addition of an alkoxylation catalyst with 2 mol of alkylene oxide, in particular 2 mol of ethylene oxide, to give the dihydroxyalkylamino group, in particular to give the dihydroxyethylamino group.

9. A process for producing polyurethane prepolymers by reacting compounds of formula 2 with a polyisocyanate and optionally with further polyols or
polyamines.

10. A process for producing polyurethane polymers by
a) reacting a compound of formula 2 with a polyisocyanate and optionally with further polyols or polyamines to give a polyurethane prepolymer, and
b) reacting the resulting polyurethane prepolymer in aqueous medium with a polyamine to give a polyurethane polymer.

11. A polyurethane prepolymer obtainable by the reaction of a compound of formula 2 with an isocyanate of the formula X(NCO)ₚ, in which p is a number from ' 2 to 4 and X is an aliphatic, cycloaliphatic, aromatic or araliphatic hydrocarbon radical.

12. The polyurethane polymer obtainable by the reaction of a compound of formula 2 with an isocyanate of the formula X(NCO)ₚ, in which p is a number from 2 to 4 and X is an aliphatic, cycloaliphatic, aromatic or araliphataic hydrocarbon radical, and the subsequent reaction of the resulting polyurethane prepolymer in an aqueous medium with a polyamine of the formula Y(NH₂)_{q}, in which Y is an aliphatic, cycloaliphatic, aromatic or araliphatic hydrocarbon radical, and q is a number from 2 to 4.

13. The use of the compounds of formula 2 for producing polyurethane prepolymers by reacting the compound of formula 2 with an isocyanate of the formula X(NCO)ₚ, in which p is a number from 2 to 4 and X is an aliphatic, cycloaliphatic, aromatic or araliphatic hydrocarbon radical.

14. The use of the compounds of formula 2 for producing polyurethane polymers by reacting the compound of formula 2 with an isocyanate of the formula X(NCO)ₚ, in which p is a number from 2 to 4 and X is an aliphatic, cycloaliphatic, aromatic or araliphatic hydrocarbon radical, and the resulting polyurethane prepolymer is reacted in an aqueous medium with a polyamine of the formula Y(NH₂)_{q}, in which Y is an aliphatic, cycloaliphatic, aromatic or araliphatic hydrocarbon radical, and q is a number from 2 to 4.

## Revendications

1. Composés de formule 2 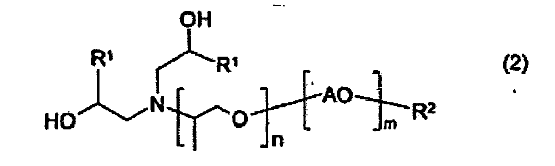 où
R¹ signifie H, méthyle ou éthyle ;
R² signifie C₁-C₄-alkyle,
A signifie un groupe C₂-C₄-alkylène,
m vaut un nombre de 1 à 400,
n vaut 1, 2, 3, 4 ou 5.

2. Composés selon la revendication 1, où au moins 50% en mole des radicaux (A-O) sont des radicaux oxyéthylène -CH₂-CH₂-O-.

3. Composés selon la revendication 2, où les autres radicaux (A-O) jusqu'à 100% en mole sont des radicaux oxypropylène -CH(CH₃)-CH₂-O-.

4. Composés selon la revendication 1, où le groupe (A-O)ₘ représente un groupe oxyéthylène pur comprenant 3 à 300 unités d'oxyéthylène.

5. Composés selon l'une ou plusieurs des revendications 1 à 4, où R¹ représente H.

6. Composés selon l'une ou plusieurs des revendications 1 à 5, où R² représente CH₃.

7. Composés selon l'une ou plusieurs des revendications 1 à 6, où -(A-O)ₘ-R² représente un groupe oxyalkylène de formule
-(CH₂-CH₂-O)_{b}-(CH(CH₃) -CH₂-O)ₐ-R² dans laquelle
a vaut un nombre de 0 à 300,
b vaut un nombre de 3 à 300, et
et R²a la signification indiquée dans la revendication 1.

8. Procédé pour la préparation de composés de formule 2, en ce qu'on prépare d'abord un α-hydroxy-Ω-alcoxypolyalkylèneglycol avec un groupe terminal hydroxy secondaire par alcoxylation de R²-OH et propoxylation consécutive, on remplace ensuite le groupe α-hydroxy secondaire par aminolyse par un groupe amino primaire et on transforme ensuite ce groupe amino primaire, sans addition d'un catalyseur d'alcoxylation, avec 2 moles d'oxyde d'alkylène, en particulier 2 moles d'oxyde d'éthylène en groupe dihydroxyalkylamino, en particulier en groupe dihydroxyéthylamino.

9. Procédé pour la préparation de prépolymères de polyuréthane, en ce qu'on transforme les composés de formule 2 avec un polyisocyanate et le cas échéant d'autres polyols ou polyamines.

10. Procédé pour la préparation de polymères de polyuréthane, en ce qu'on transforme
a) un composé de formule 2 avec un polyisocyanate et le cas échéant d'autres polyols ou polyamines en un prépolymère de polyuréthane, et
b) le prépolymère de polyuréthane ainsi obtenu en milieu aqueux avec une polyamine en polymère de polyuréthane.

11. Prépolymères de polyuréthane, pouvant être obtenus par réaction d'un composé de formule 2 avec un isocyanate de formule X(NCO)ₚ, où p représente un nombre de 2 à 4 et X représente un radical hydrocarboné aliphatique, cycloaliphatique, aromatique ou araliphatique.

12. Polymères de polyuréthane, qui peuvent être obtenus par la réaction d'un composé de formule 2, avec un isocyanate de formule X(NCO)ₚ, où p représente un nombre de 2 à 4 et X représente un radical hydrocarboné aliphatique, cycloaliphatique, aromatique ou araliphatique et la transformation consécutive du prépolymère de polyuréthane ainsi obtenu en milieu aqueux avec une polyamine de formule Y(NH₂)_{q}, où Y représente un radical hydrocarboné aliphatique, cycloaliphatique, aromatique ou araliphatique et q représente un nombre de 2 à 4.

13. Utilisation des composés de formule 2 pour la préparation de prépolymères de polyuréthane, en ce qu'on fait réagir le composé de formule 2 avec un isocyanate de formule X(NCO)ₚ, où p représente un nombre de 2 à 4 et X représente un radical hydrocarboné aliphatique, cycloaliphatique, aromatique ou araliphatique.

14. Utilisation de composés de formule 2 pour la préparation de polymères de polyuréthane, en ce qu'on fait réagir le composé de formule 2 avec un isocyanate de formule X(NCO)ₚ, où p représente un nombre de 2 à 4 et X représente un radical hydrocarboné aliphatique, cycloaliphatique, aromatique ou araliphatique et on transforme le prépolymère de polyuréthane ainsi obtenu en milieu aqueux avec une polyamine de formule Y(NH₂)_{q}, où Y représente un radical hydrocarboné aliphatique, cycloaliphatique, aromatique ou araliphatique et q représente un nombre de 2 à 4.
